# EUROPEAN PATENT APPLICATION

(11) **EP 4 775 944 A1**
(43) Date of publication of application: **15.07.2026**
(21) Application number: 25227089.7
(22) Date of filing: 23.12.2025
(51) Int. Cl.: G01L 19/00, A61M 1/36, G01L 19/14

(54) **PRESSURE DETECTION DEVICE**

(30) Priority: 10.01.2025 JP 2025004136
(71) Applicant: Surpass Industry Co., Ltd., Gyoda-shi Saitama 361-0037 (JP)
(72) Inventor: HASUNUMA, Masahiro, Gyoda-shi, 361-0037 (JP); TAKADA, Kouki, Gyoda-shi, 361-0037 (JP)
(74) Representative: Patentwerk B.V.

(57) **Abstract**

Provided is a pressure detection device in which a pressure detection unit includes a first connecting part that is a magnet, and the flow path unit 20 includes a resin pressure receiving diaphragm 22 and a second connecting part 23 having a magnetic material 23b. In a state where the flow path unit 20 is attached to the pressure detection unit, the first connecting part and the second connecting part 23 are arranged attracted to each other by magnetic force. The second connecting part 23 includes a resin joining member 23a in which a first joining face 23a1 joined to the pressure receiving diaphragm 22 and a second joining face 23a2 joined to the magnetic material 23b are formed at both ends in a direction along an axis Y2. The first joining face 23a1 of the joining member 23a and the pressure receiving diaphragm 22 are joined to each other by an adhesive agent, and the second joining face 23a2 of the joining member 23a and the magnetic material 23b are joined to each other by an adhesive agent.

## Description

### BACKGROUND

### 1. TECHNICAL FIELD

The present invention relates to a coupling device.

### 2. DESCRIPTION OF RELATED ART

Pressure detection devices configured such that a flow path to which a fluid is introduced is formed in a flow path unit, the flow path unit is attached to a pressure detection unit, and the pressure of the fluid flowing through the flow path unit is detected by the pressure detection unit are conventionally known. In a pressure detection device disclosed in Japanese Patent Application Laid-Open No. 2018-136216, a first connecting part formed of a magnet is provided to a diaphragm of a pressure detection unit, and a second connecting part formed of a magnetic material is provided to a diaphragm of a flow path unit.

In the pressure detection device disclosed in Japanese Patent Application Laid-Open No. 2018-136216, the first connecting part and the second connecting part are arranged attracted to each other by magnetic force in a state where the flow path unit has been attached to the pressure detection unit. Thus, when the pressure of a fluid flowing through the flow path is a negative pressure, the second connecting part is pulled to the flow path side by the pressure of the fluid, and the second connecting part pulls the first connecting part connected thereto by magnetic force to the flow path side. Accordingly, the pressure of the fluid can be detected as a negative pressure by the pressure detection part.

In the pressure detection device disclosed in Japanese Patent Application Laid-Open No. 2018-136216, the second connecting part formed of a magnetic material is joined to a flat face on the pressure detection unit side of the diaphragm of a flow path unit by an adhesive agent.

However, since the second connecting part is joined to the flat face, deformation of the diaphragm due to a change in the pressure of a fluid causes a change in the stress in the joining portion between the flat face and the second connecting part. Repetition of such operation causing diaphragm deformation then causes deterioration of the joining portion, this causes the second connecting part to separate from the diaphragm, and this makes it no longer possible to properly detect the pressure of the fluid.

### SUMMARY

The present invention has been made in view of such circumstances and intends to, in a pressure detection device including a flow path unit and a pressure detection unit, prevent a connecting part joined to a pressure receiving diaphragm of the flow path unit from separating from the pressure receiving diaphragm.

The present invention employs the following solutions in order to achieve the object described above.

The pressure detection device according to the first aspect of the present invention includes: a pressure detection unit configured to determine a pressure transferred to a pressure detection part; a flow path unit having a flow path formed in the flow path unit, a fluid being allowed to flow through the flow path; and an attaching mechanism configured to detachably attach the flow path unit to the pressure detection unit. The pressure detection unit includes a pressure sensor having the pressure detection part, and a first connecting part joined to the pressure detection part. The flow path unit includes a resin pressure receiving part configured to displace in response to receiving a pressure of a fluid flowing through the flow path, and a second connecting part joined to the pressure receiving part. Either one of the first connecting part and the second connecting part includes a magnet, and the other of the first connecting part and the second connecting part includes a magnet or a magnetic material. In a state where the flow path unit is attached to the pressure detection unit by the attaching mechanism, the first connecting part and the second connecting part are arranged attracted to each other by magnetic force. The pressure receiving part is formed circularly in planar view and formed in a plate shape from the center position to an end. The second connecting part includes a resin joining member formed cylindrically extending along the axis and having a first joining face and a second joining face formed at both ends in a direction along the axis, the first joining face being joined to the pressure receiving part, and the second joining face being joined to the magnet or the magnetic material The first joining face of the joining member and the pressure receiving part are joined to each other by an adhesive agent in a predetermined range surrounding the center position, and the second joining face of the joining member and the magnet or the magnetic material are joined to each other by an adhesive agent.

According to the pressure detection device of the first aspect of the present invention, in a state where the flow path unit has been attached to the pressure detection unit by the attaching mechanism, the first connecting part joined to the pressure detection part and the second connecting part joined to the pressure receiving part are arranged attracted to each other by magnetic force. Thus, when the pressure of a fluid flowing through the flow path is a positive pressure, the second connecting part joined to the pressure receiving part is separated from the flow path side by the pressure of the fluid, and the second connecting part causes the first connecting part to be pushed against the pressure detection part. Accordingly, the pressure of the fluid is detected as a positive pressure by the pressure detection part.

Further, when the pressure of a fluid flowing through the flow path is a negative pressure, the second connecting part joined to the pressure receiving part by the pressure of the fluid is pulled to the flow path side by the pressure of the fluid, and the second connecting part pulls the first connecting part, which is connected thereto by magnetic force, to the flow path side. Accordingly, the pressure of the fluid is detected as a negative pressure by the pressure detection part. As described above, according to the pressure detection device of the present aspect, the pressure of a fluid can be accurately determined in both cases where the pressure of the fluid is a positive pressure and a negative pressure while enhancing speed and safety of operation to change the fluid that is to be introduced to the flow path.

Further, according to the pressure detection device of the first aspect of the present invention, the first joining face of the joining member joined to the pressure receiving part and the pressure receiving part are joined to each other by an adhesive agent in a predetermined range surrounding the center position. Since the joining member and the pressure receiving part are made of resin, respectively, the joining member and the pressure receiving part are joined to each other more firmly than in a case where a magnet or a magnetic material that is a metal material is joined to the pressure receiving part by an adhesive agent. Thus, even when a change in the stress occurs in the joining portion between the second connecting part and the pressure receiving part, it is possible to maintain the state where the second connecting part is firmly joined to the pressure receiving part and prevent the second connecting part joined to the pressure receiving part of the flow path unit from separating from the pressure receiving part.

Further, according to the pressure detection device of the first aspect of the present invention, the second joining face of the joining member and the magnet or the magnetic material that is a metal material are joined to each other by an adhesive agent. Since the magnet or the magnetic material is made of metal while the joining member is made of resin, the joining strength between the second joining face and the metal material is lower than in a case where resin members are joined to each other. In contrast, the metal material is not directly joined to the pressure receiving part, and a displacement of the second joining face is small even when receiving a pressure of a fluid. It is thus possible to suitably maintain the state where the second joining face and the magnet or the magnetic material, which is a metal material, are joined to each other by an adhesive agent.

The pressure detection device according to the second aspect of the present invention further has the following configuration in the first aspect. That is, the joining member includes a communication through-hole arranged at the center position, the first joining face and the second joining face being in communication with each other through the communication through-hole.

According to the pressure detection device of the second aspect of the present invention, an excessive portion of the adhesive agent applied to the first joining face and the second joining face can be accommodated in the communication through-hole, and a state where the first joining face and the second joining face are joined to each other by a suitable amount of the adhesive agent can be obtained.

The pressure detection device according to the third aspect of the present invention further has the following configuration in the first aspect. That is, a first outer diameter of the first joining face when viewed in planar view is set to be 1/3 times or greater and 1/2 times or less of a second outer diameter of a region of the pressure receiving part when viewed in planar view, the fluid coming into contact with the region.

According to the pressure detection device of the third aspect of the present invention, the first outer diameter of the first joining face is 1/3 times or greater of the second outer diameter of the region of the pressure receiving part with which a fluid comes into contact, and this can ensure a sufficient size of the first outer diameter of the first joining face joined to the pressure receiving part and thereby ensure sufficient attractive force so that the second connecting part and the first connecting part are attracted to each other by magnetic force. Further, the first outer diameter of the first joining face is 1/2 times or less of the second outer diameter of the region of the pressure receiving part with which a fluid comes into contact, and this makes it possible to prevent the ability to follow a pressure change of the pressure receiving part from compromising due to an excessively larger first outer diameter of the first joining face.

The pressure detection device according to the fourth aspect of the present invention further has the following configuration in the first aspect. That is, the outer diameter of the first connecting part about the axis orthogonal to the pressure receiving part is larger than the outer diameter of the second connecting part about the axis.

According to the pressure detection device of the fourth aspect of the present invention, it is possible to ensure a sufficient outer diameter of the first connecting part and thereby ensure sufficient attractive force so that the second connecting part and the first connecting part are attracted to each other by magnetic force.

The pressure detection device according to the fourth aspect of the present invention further has the following configuration in any one of the first aspect to the fourth aspect. That is, the first joining face is a flat face arranged on a plane orthogonal to the axis, the second joining face is a stepwise face having a recess formed at the center position, the magnet or the magnetic material joined to the joining member includes a protruding part formed at the center position, and the joining member is joined to the magnet or the magnetic material with the protruding part being inserted in the recess.

According to the pressure detection device of the fifth aspect of the present invention, since the joining member is joined to the magnet or the magnetic material with the protruding part being inserted in the recess, when joining the joining member to the magnet or the magnetic material, it is possible to suitably prevent these members from being shifted in a radial direction orthogonal to the axis and enhance the joining strength of these members.

According to the present invention, in a pressure detection device including a flow path unit and a pressure detection unit, it is possible to prevent a connecting part joined to a pressure receiving diaphragm of the flow path unit from separating from the pressure receiving diaphragm.

### BRIEF DESCRIPTION OF THE DRAWINGS

Fig. 1 is a front view illustrating a pressure detection device of one embodiment of the present invention.
Fig. 2 is a diagram illustrating a state where a flow path unit has been removed from the pressure detection device illustrated in Fig. 1.
Fig. 3 is an arrow A-A sectional view illustrating the state where the flow path unit has been removed from the pressure detection device illustrated in Fig. 1.
Fig. 4 is a bottom view of the flow path unit illustrated in Fig. 3.
Fig. 5 is an arrow A-A sectional view of the pressure detection device illustrated in Fig. 1.
Fig. 6 is a partial enlarged view of the flow path unit illustrated in Fig. 5.
Fig. 7 is a flowchart illustrating a manufacturing method of the pressure detection device.
Fig. 8 is a sectional view illustrating a state before a joining member and a magnetic material are adhered to each other.
Fig. 9 is a sectional view illustrating a state where a connecting part has been installed to a joining jig.
Fig. 10 is a sectional view illustrating a state where the connecting part and a pressure receiving diaphragm are irradiated with a UV ray.
Fig. 11 is a sectional view illustrating a state where the pressure receiving diaphragm has been joined to a flow path body.
Fig. 12 is a sectional view illustrating a state where a nut has been attached to the flow path body.
Fig. 13 is a sectional view illustrating a state where the pressure receiving diaphragm of the flow path unit of the present embodiment is deformed.
Fig. 14 is a sectional view illustrating a state where a pressure receiving diaphragm of a flow path unit of a comparative example is deformed.

### DETAILED DESCRIPTION

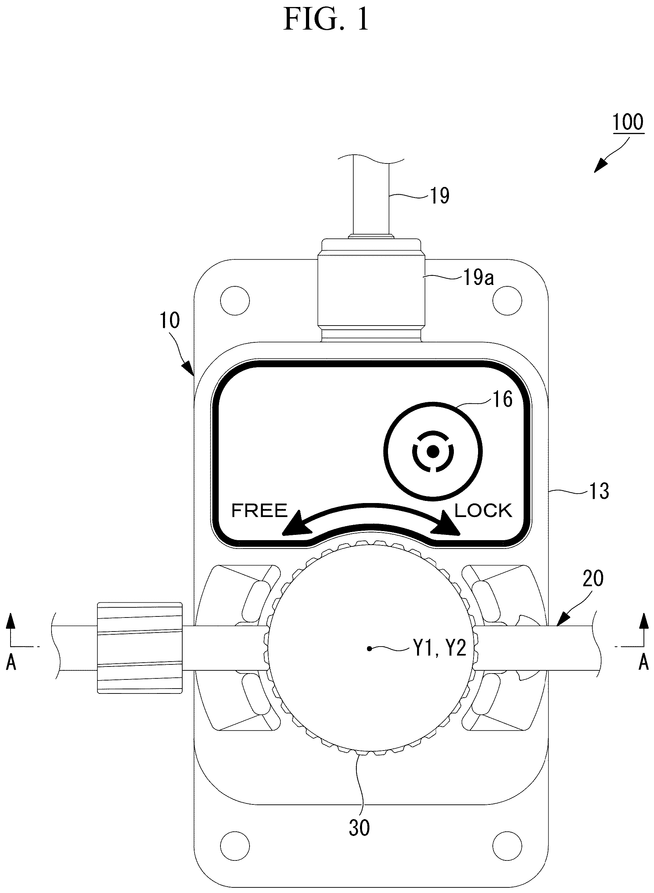

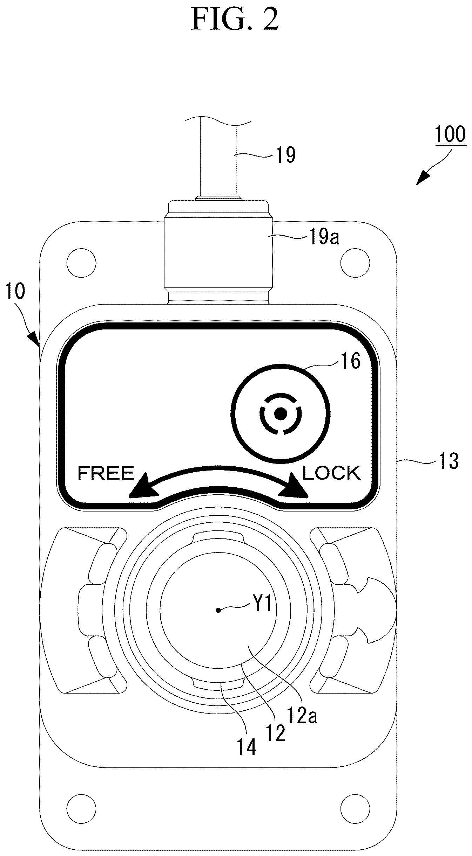

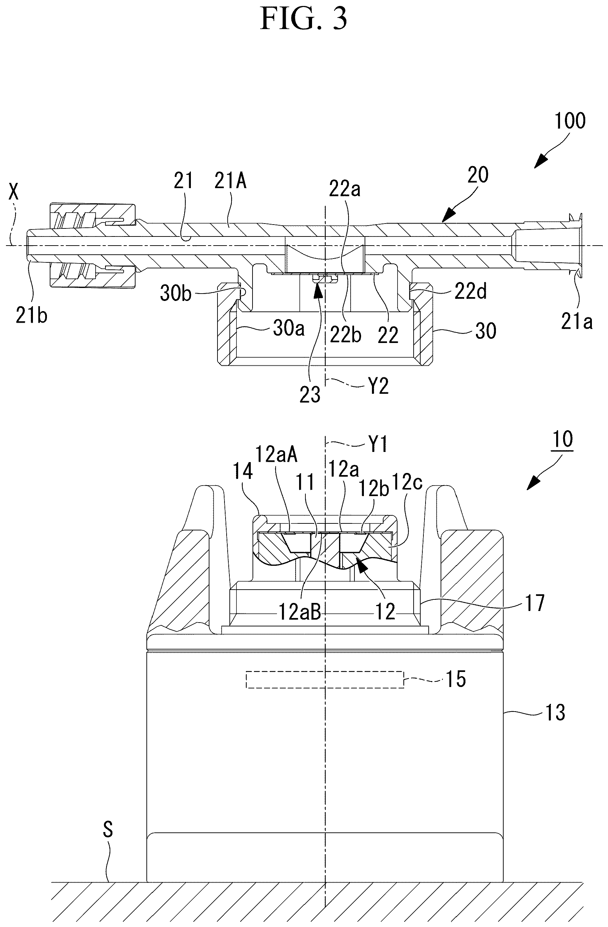

A pressure detection device 100 of one embodiment of the present invention will be described below with reference to the drawings. Fig. 1 is a front view illustrating the pressure detection device 100 of one embodiment of the present invention. Fig. 2 is a diagram illustrating a state where a flow path unit 20 has been removed from the pressure detection device 100 illustrated in Fig. 1. Fig. 3 is an arrow A-A sectional view illustrating the state where the flow path unit 20 has been removed from the pressure detection device 100 illustrated in Fig. 1.

As illustrated in Fig. 1, the pressure detection device 100 of the present embodiment includes a pressure detection unit 10 attached to an installation face S by a fastening bolt (not illustrated), the flow path unit 20 inside which a flow path 21 through which a fluid is allowed to flow is formed, and a nut 30 (attaching mechanism) configured to detachably attach the flow path unit 20 to the pressure detection unit 10.

As illustrated in Fig. 1, the pressure detection unit 10 is attached to the installation face S, and the flow path unit 20 is attached to the pressure detection unit 10 by the nut 30. The pressure detection device 100 is attached to the installation face S with the flow path unit 20 being attached to and integrated into the pressure detection unit 10 by the nut 30.

An inflow-side pipe (not illustrated) through which a fluid is allowed to flow into an inflow port 21a is attached to the inflow port 21a of the flow path unit 20 illustrated in Fig. 3, and an outflow-side pipe (not illustrated) through which a fluid flowing out of an outflow port 21b is allowed to flow is attached to the outflow port 21b of the flow path unit 20. The pressure of a fluid flowing through a flow path 21 from the inflow port 21a to the outflow port 21b is detected by the pressure detection unit 10. Herein, the fluid is, for example, a liquid such as blood or a dialysis solution.

As illustrated in Fig. 3, the pressure detection unit 10 includes a body 13 attached to the installation face S. As illustrated in Fig. 2 and Fig. 3, a cable 19 electrically connecting a pressure sensor 12 arranged inside and an external control device (not illustrated) to each other is attached to the body 13 of the pressure detection unit 10 via a cable attaching nut 19a.

Next, the pressure detection unit 10 will be described in detail with reference to Fig. 1 to Fig. 3. The pressure detection unit 10 illustrated in Fig. 1 to Fig. 3 is a device configured to detect a pressure transferred to a diaphragm (pressure detection part) 12a. The pressure detection unit 10 includes a connecting part (first connecting part) 11, the pressure sensor 12, the body 13 inside which the pressure sensor 12 is arranged, a sensor holding part 14 that holds the pressure sensor 12 at the body 13, a sensor circuit board 15 used for transferring power supply and electrical signals between the pressure sensor 12 and the cable 19, and a zero-point adjustment switch 16 used for performing zero-point adjustment of the pressure sensor 12.

The connecting part 11 is a permanent magnet formed cylindrically along the axis Y1 and is, for example, formed of neodymium or the like. The connecting part 11 is joined to a second face 12aB of the diaphragm 12a of the pressure sensor 12 by an adhesive agent (for example, an epoxy resin based adhesive agent). As illustrated in Fig. 3, the end face of the connecting part 11 joined to the diaphragm 12a is formed in a planar shape arranged on a plane orthogonal to the axis Y1. The connecting part 11 pulls a connecting part (second connecting part) 23, which is a magnetic material, by magnetic force of the permanent magnet and maintains the state where these connecting parts are in contact with each other. The connecting part 23 of the flow path unit 20 will be described later.

Note that, although the connecting part 11 includes a permanent magnet and the connecting part 23 includes a magnetic material in the above description, other aspects may be employed. For example, both the connecting part 11 and the connecting part 23 may include permanent magnets. Alternatively, the connecting part 11 may include a magnetic material, and the connecting part 23 may include a permanent magnet. As discussed above, in the pressure detection device 100 of the present embodiment, either one of the connecting part 11 and the connecting part 23 includes a magnet, and the other of the connecting part 11 and the connecting part 23 includes a magnet or a magnetic material. In the following, an example in which the connecting part 11 includes a permanent magnet and the connecting part 23 includes a magnetic material will be described.

As illustrated in Fig. 3, the pressure sensor 12 includes the diaphragm 12a formed of an anti-corrosive material (for example, sapphire) as a thin film, a distortion resistive part 12b joined to the second face 12aB of the diaphragm 12a, and a base part 12c that holds the diaphragm 12a.

The pressure sensor 12 is a distortion type sensor that outputs a pressure signal in accordance with a change in the resistance of the distortion resistive part 12b that is deformed in accordance with a pressure transferred from the connecting part 23 to a first face 12aA of the diaphragm 12a. A through-hole penetrating the diaphragm 12a is formed in the base part 12c, and the second face 12aB of the diaphragm 12a is maintained at the atmospheric pressure. Thus, the pressure sensor 12 is a sensor that detects a gauge pressure based on the atmospheric pressure as a reference. As illustrated in Fig. 2, in a state where the flow path unit 20 is not attached to the pressure detection unit 10, the diaphragm 12a of the pressure sensor 12 remains to be exposed to the outside.

The sensor holding part 14 is a member formed cylindrically about the axis Y1. The sensor holding part 14 has, at the top end, the inner diameter smaller than the outer diameter of the pressure sensor 12 and thus can hold the pressure sensor 12 so that the pressure sensor 12 does not come off upward. The sensor holding part 14 holds the diaphragm 12a joined to the base part 12c by an adhesive agent (adhesive glass).

The sensor circuit board 15 includes an amplifier circuit (not illustrated) that amplifies a pressure signal output by the pressure sensor 12, an interface circuit that transfers a pressure signal amplified by the amplifier circuit to a pressure signal line (not illustrated) of the cable 19, a power supply circuit (not illustrated) that transfers, to the pressure sensor 12, a power supply voltage externally supplied via the cable 19, a zero-point adjustment circuit (not illustrated) that performs zero point adjustment when the zero-point adjustment switch 16 is pressed, and the like. The zero-point adjustment circuit is a circuit that, when the zero-point adjustment switch 16 is pressed, performs adjustment so that a pressure signal output by the pressure sensor 12 at this time is set as a reference value (for example, zero).

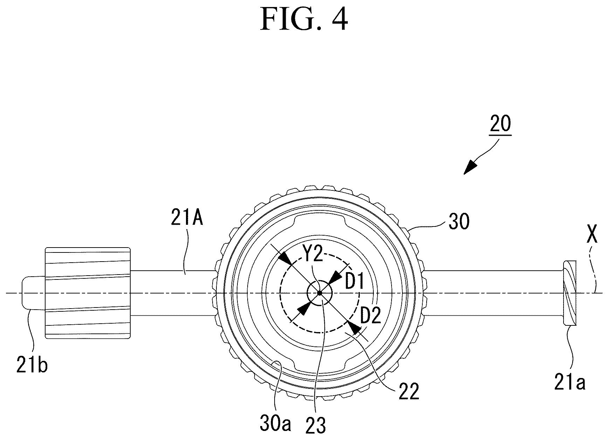

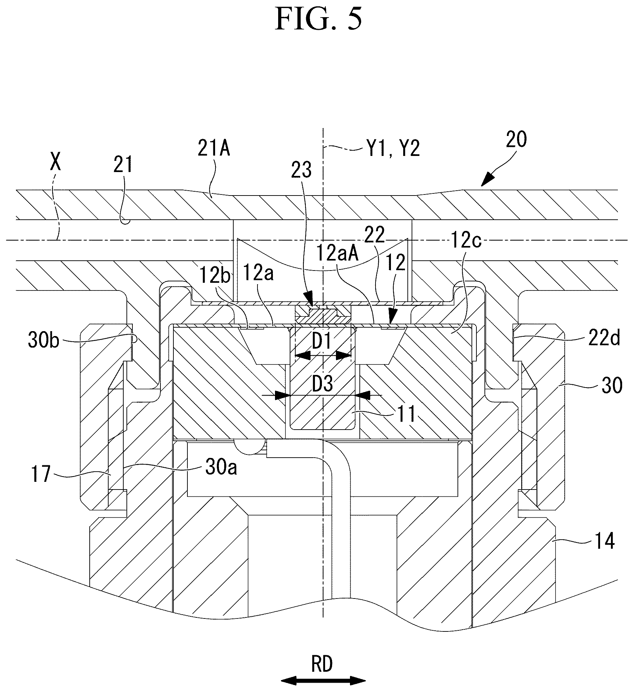

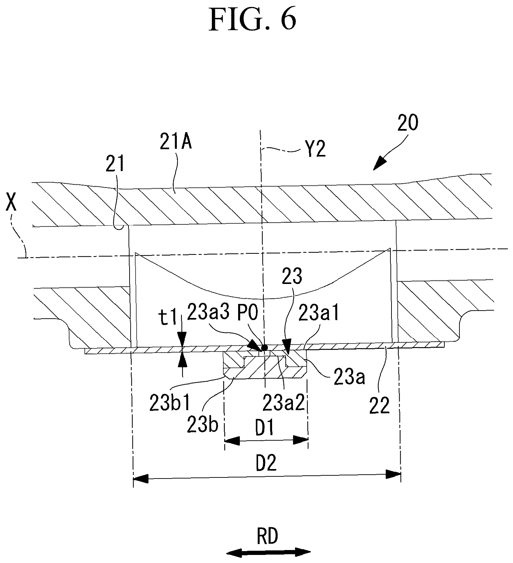

Next, the flow path unit 20 will be described in detail with reference to Fig. 3 to Fig. 6. Fig. 4 is a bottom view of the flow path unit 20 illustrated in Fig. 3. Fig. 5 is an arrow A-A sectional view of the pressure detection device 100 illustrated in Fig. 1. Fig. 6 is a partial enlarged view of the flow path unit 20 illustrated in Fig. 5.

As illustrated in Fig. 3, the flow path unit 20 includes a flow path body 21A inside which the flow path 21 through which a fluid is allowed to flow in the flow direction extending along the axis X from the inflow port 21a to the outflow port 21b is formed, a pressure receiving diaphragm 22 that displaces in response to receiving the pressure of the fluid flowing through the flow path 21 at the first face 22a, and a connecting part (second connecting part) 23 joined to the second face 22b of the pressure receiving diaphragm 22.

The pressure receiving diaphragm 22 is a member formed of an anti-corrosive resin material (for example, polycarbonate) as a thin film. The pressure receiving diaphragm 22 is a member formed circularly in planar view about the axis Y2 as the central axis, and the outer circumferential edge thereof is joined to the flow path 21 by adhesion or welding. The pressure receiving diaphragm 22 is formed as a thin film and is thus deformed in a direction along the axis Y2 by the pressure of a fluid flowing through the flow path 21. As illustrated in Fig. 6, the pressure receiving diaphragm 22 is formed as a plate with a constant thickness t1 from the center position P0 through which the axis Y2 passes to the end.

The connecting part 23 is a member formed cylindrically along the axis Y2 orthogonal to the pressure receiving diaphragm 22. As illustrated in Fig. 4, the connecting part 23 is formed circularly in planar view. As illustrated in Fig. 5, the connecting part 23 is arranged attracted to the connecting part 11, which is a permanent magnet, by the magnetic force thereof in a state where the flow path unit 20 has been attached to the pressure detection unit 10 by the nut 30. As illustrated in Fig. 6, the connecting part 23 includes a joining member 23a and a magnetic material 23b.

The joining member 23a is a member made of resin (for example, made of polyvinyl chloride (PVC)) in which a first joining face 23a1 joined to the pressure receiving diaphragm 22 and a second joining face 23a2 joined to the magnetic material 23b are formed at both ends in a direction along the axis Y2. The magnetic material 23b is formed of an iron material such as S45C defined by the JIS standard.

The first joining face 23a1 of the joining member 23a and the pressure receiving diaphragm 22 are joined to each other by an adhesive agent in a range of a first outer diameter D1 surrounding the center position P0. Further, the second joining face 23a2 of the joining member 23a and the magnetic material 23b are joined to each other by an adhesive agent in a range of the first outer diameter D1 surrounding the center position P0.

The joining member 23a includes a communication through-hole 23a3 through which the first joining face 23a1 and the second joining face 23a2 are in communication with each other and that is arranged at the center position P0. The communication through-hole 23a3 accommodates an excessive portion of the adhesive agent applied to the first joining face 23a1 and the second joining face 23a2, and this makes it possible to obtain a state where the first joining face 23a1 and the second joining face 23a2 are joined to each other by a suitable amount of the adhesive agent.

As illustrated in Fig. 4 and Fig. 6, the outer diameter of the first joining face 23a1 when viewed in planar view is the first outer diameter D1, and the outer diameter of the region of the pressure receiving diaphragm 22 with which the fluid comes into contact when viewed in planar view is the second outer diameter D2. The first outer diameter D1 is set to be 1/3 times or greater and 1/2 times or less of the second outer diameter D2.

A C-surface 23b1 is formed to the end in a direction along the axis Y2 of the magnetic material 23b. The C-surface 23b1 is a chamfered portion formed in an annular shape circumferentially about the axis Y2 to the end in the radial direction RD orthogonal to the axis Y2.

As illustrated in Fig. 5, in the state where the flow path unit 20 has been attached to the pressure detection unit 10, the connecting part 23 of the flow path unit 20 is in contact with the first face 12aA of the diaphragm 12a of the pressure detection unit 10. The connecting part 23 transfers, to the diaphragm 12a, the pressure of a fluid flowing through the flow path 21.

As illustrated in Fig. 5, in the state where the flow path unit 20 has been attached to the pressure detection unit 10, the axis Y1 and the axis Y2 are arranged at the same position in the radial direction RD. As illustrated in Fig. 5, the third outer diameter D3 of the connecting part 11 about the axis Y1 is larger than the first outer diameter D1 of the connecting part 23 about the axis Y2. By ensuring a sufficient third outer diameter D3 of the connecting part 11 relative to the first outer diameter D1 of the connecting part 23, it is possible to ensure sufficient attractive force so that the connecting part 23 and the connecting part 11 are attracted to each other by magnetic force.

Next, the structure for attaching the flow path unit 20 to the pressure detection unit 10 by the nut 30 will be described. As illustrated in Fig. 3, an annular groove 22d extending circumferentially about the axis Y2 is formed in the outer circumferential face on the bottom end side of the flow path unit 20. On the other hand, an annular protrusion 30b extending circumferentially about the axis Y2 is formed in the inner circumferential face of the nut 30. The nut 30 formed of an elastically deformable material (for example, a resin material) is pushed into the annular groove 22d, and thereby the annular protrusion 30b is in engagement with the annular groove 22d.

In the state where the annular protrusion 30b is in engagement with the annular groove 22d as illustrated in Fig. 3, a slight clearance is provided between the outer circumferential face of the annular protrusion 30b and the inner circumferential face of the annular groove 22d. Thus, the nut 30 is rotatable relatively about the axis Y2 when attached to the pressure detection unit 10. This enables the operator to rotate the nut 30 about the axis Y2 while fixing the pressure detection unit 10 to the installation face S.

As illustrated in Fig. 3, the nut 30 is a circular, annular member in which an internal thread 30a extending circumferentially about the axis Y2 is formed in the inner circumferential face thereof. The nut 30 is a mechanism that detachably attaches the flow path unit 20 to the pressure detection unit 10 by fastening the internal thread 30a into an external thread 17 formed on the pressure detection unit 10 or by releasing the fastening.

The operator fastens the internal thread 30a of the nut 30 and the external thread 17 of the pressure detection unit 10 to each other by rotating the nut 30 in the fastening direction (direction indicated by "LOCK" in Fig. 1, Fig. 2) about the axis Y1 while gripping the flow path unit 20. As the internal thread 30a of the nut 30 and the external thread 17 of the pressure detection unit 10 are fastened to each other, the connecting part 23 gradually comes close to the first face 12aA of the diaphragm 12a and finally comes into contact with the first face 12aA of the diaphragm 12a, and this results in the state illustrated in Fig. 5.

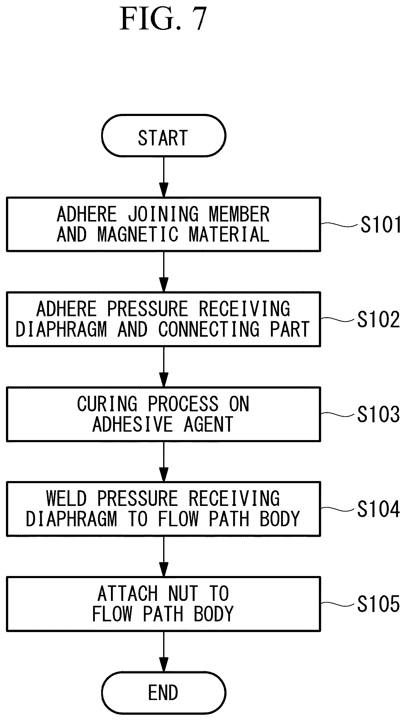

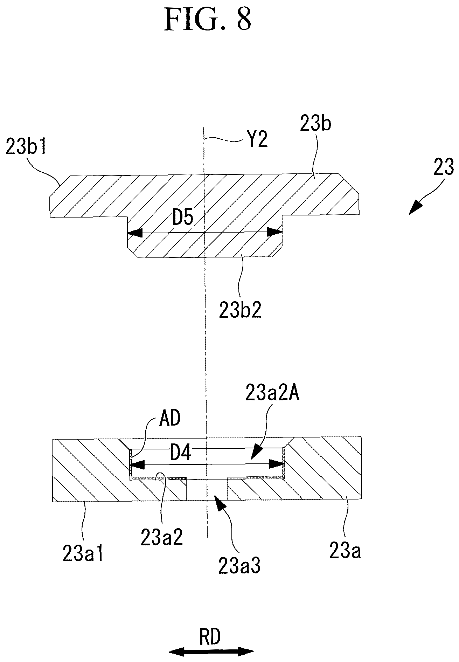

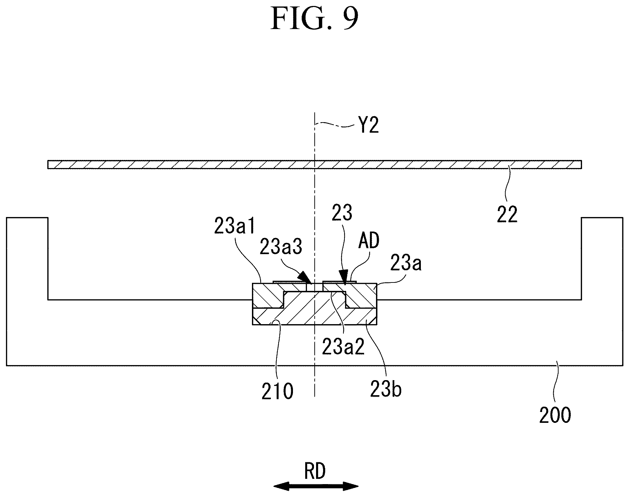

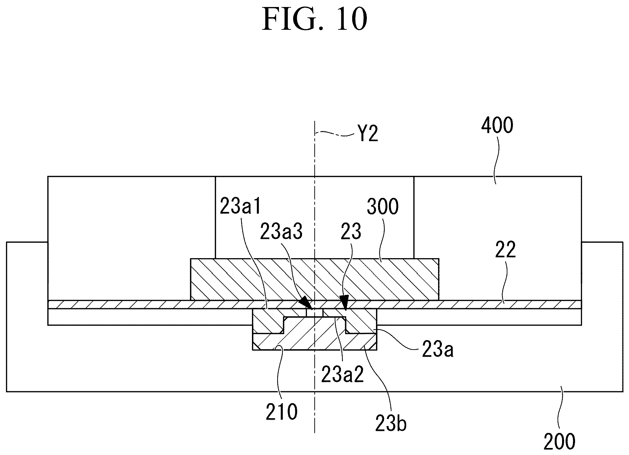

Next, a manufacturing method of the pressure detection device 100 according to one embodiment of the present invention will be described. Fig. 7 is a flowchart illustrating the manufacturing method of the pressure detection device 100. Fig. 8 is a sectional view illustrating a state before the joining member 23a and the magnetic material 23b are adhered to each other. Fig. 9 is a sectional view illustrating a state where the connecting part 23 has been installed to a joining jig 200. Fig. 10 is a sectional view illustrating a state where the connecting part 23 and the pressure receiving diaphragm 22 are irradiated with a UV ray.

In step S101, the operator adheres the joining member 23a and the magnetic material 23b to each other. As illustrated in Fig. 8, the first joining face 23a1 of the joining member 23a is a flat face arranged on the plane orthogonal to the axis Y2. The second joining face 23a2 of the joining member 23a is a stepwise face in which a recess 23a2A is formed at the center position in the radial direction RD.

In the magnetic material 23b, the protruding part 23b2 is formed at the center position in the radial direction RD. The outer diameter D5 of the protruding part 23b2 is set slightly smaller than the inner diameter D4 of the recess 23a2A of the joining member 23a. The operator applies an adhesive agent AD to the recess 23a2A of the second joining face 23a2 of the joining member 23a and inserts the protruding part 23b2 of the magnetic material 23b into the recess 23a2A of the joining member 23a to adhere the joining member 23a to the magnetic material 23b. The adhesive agent AD is, for example, a UV-curable adhesive agent.

In step S102, the operator adheres the pressure receiving diaphragm 22 and the connecting part 23 to each other. As illustrated in Fig. 9, the joining jig 200 has a recess 210 for installing the connecting part 23 therein. The operator grips and then installs the connecting part 23 in the recess 210 of the joining jig 200. The operator then applies the adhesive agent AD to the first joining face 23a1 of the joining member 23a. Finally, the operator moves the pressure receiving diaphragm 22 while matching the center position of the pressure receiving diaphragm 22 with the center position of the connecting part 23 and adheres the pressure receiving diaphragm 22 to the connecting part 23.

In step S103, the operator performs a curing process of the adhesive agent AD into a state where the joining member 23a and the magnetic material 23b of the connecting part 23 are joined to each other and the joining member 23a and the pressure receiving diaphragm 22 are joined to each other. As illustrated in Fig. 10, the operator arranges a glass plate 300 having an outer diameter larger than the connecting part 23 to come into contact with the center position of the pressure receiving diaphragm 22. Further, the operator installs, on the glass plate 300, a holding member 400 that provides a load for holding a state where the glass plate 300 is in contact with the pressure receiving diaphragm 22.

The operator operates an irradiation device (not illustrated) that emits a UV ray to the center position of the pressure receiving diaphragm 22 from above the glass plate 300 in the state illustrated in Fig. 10 to emit a UV ray to the center position of the pressure receiving diaphragm 22 for a predetermined time. The adhesive agent AD applied between the joining member 23a and the magnetic material 23b of the connecting part 23 and the adhesive agent AD applied between the joining member 23a and the pressure receiving diaphragm 22 are cured by the UV ray. This results in a state where the joining member 23a and the magnetic material 23b of the connecting part 23 are joined to each other, and the joining member 23a and the pressure receiving diaphragm 22 are joined to each other.

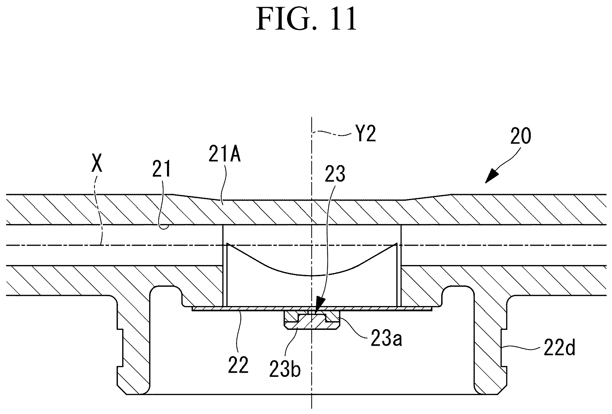

In step S104, the operator removes the pressure receiving diaphragm 22 from the joining jig 200 and welds the entire circumference of the end of the pressure receiving diaphragm 22 to the flow path body 21A to obtain the state illustrated in Fig. 11. Fig. 11 is a sectional view illustrating a state where the pressure receiving diaphragm 22 has been joined to the flow path body 21A. For the welding between the pressure receiving diaphragm 22 and the flow path body 21A, for example, an ultrasonic welding apparatus (not illustrated) is used that oscillates the pressure receiving diaphragm 22 by using ultrasonic waves to generate frictional heat at the interface between the pressure receiving diaphragm 22 and the flow path body 21A.

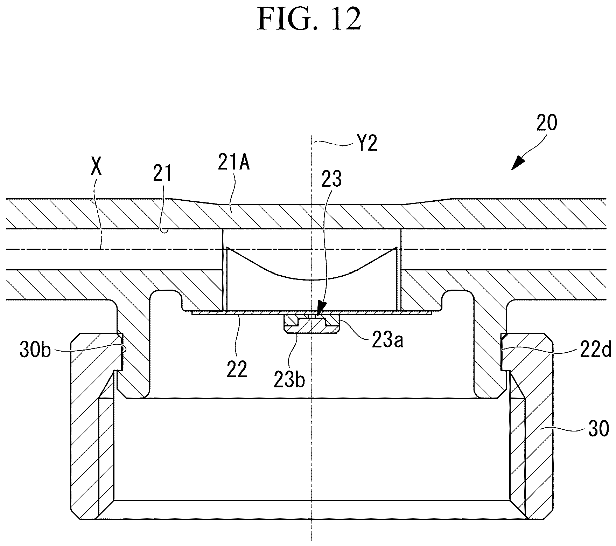

In step S105, the operator attaches the nut 30 to the flow path body 21A. The operator pushes the nut 30 into the annular groove 22d and partially elastically deforms the flow path body 21A to engage the annular protrusion 30b with the annular groove 22d to have a state illustrated in Fig. 12. Fig. 12 is a sectional view illustrating the state where the nut 30 has been attached to the flow path body 21A.

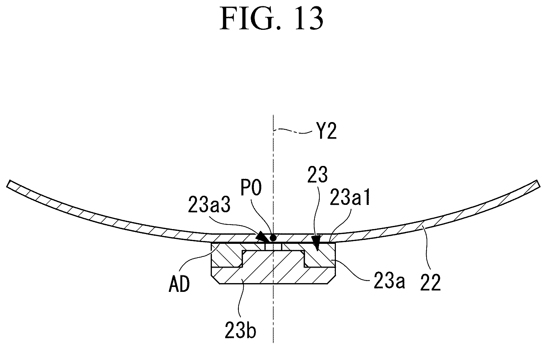

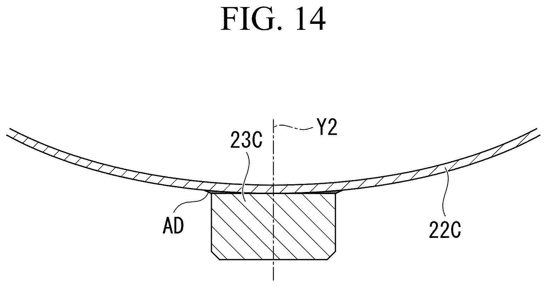

Herein, the joining state between the connecting part 23 and the pressure receiving diaphragm 22 when the pressure receiving diaphragm 22 is deformed will be described with reference to Fig. 13 and Fig. 14. Fig. 13 is a sectional view illustrating a state where the pressure receiving diaphragm 22 of the flow path unit 20 of the present embodiment is deformed. Fig. 14 is a sectional view illustrating a state where a pressure receiving diaphragm 22C of a flow path unit of a comparative example is deformed.

As illustrated in Fig. 13, the resin pressure receiving diaphragm 22 of the present embodiment is joined to the resin joining member 23a by the adhesive agent AD and thus is firmly joined to the joining member 23a compared to a case of being joined to a metal material by the adhesive agent AD. Therefore, a state where the pressure receiving diaphragm 22 and the connecting part 23 are firmly joined to each other is maintained even when the pressure receiving diaphragm 22 is deformed.

In contrast, as illustrated in Fig. 14, since the resin pressure receiving diaphragm 22C of the comparative example is joined to the connecting part 23C, which is a metal material, by the adhesive agent AD, the joining strength to the connecting part 23C is reduced compared to a case of being joined to the resin joining member 23a by the adhesive agent AD. Thus, repetition of deformation of the pressure receiving diaphragm 22C is likely to cause a reduction in the joining force at the portion where the pressure receiving diaphragm 22C and the connecting part 23C are in contact with each other.

The effects and advantages achieved by the pressure detection device 100 of the present embodiment described above will be described.

According to the pressure detection device 100 of the present embodiment, in a state where the flow path unit 20 has been attached to the pressure detection unit 10 by the nut 30, the connecting part 11 joined to the diaphragm 12a and the connecting part 23 joined to the pressure receiving diaphragm 22 are arranged attracted to each other by magnetic force. Thus, when the pressure of a fluid flowing through the flow path 21 is a positive pressure, the connecting part 23 joined to the pressure receiving diaphragm 22 is separated from the flow path 21 side by the pressure of the fluid, and the connecting part 23 causes the connecting part 11 to be pushed against the diaphragm 12a. Accordingly, the pressure of the fluid is detected as a positive pressure by the diaphragm 12a.

Further, when the pressure of a fluid flowing through the flow path 21 is a negative pressure, the connecting part 23 joined to the pressure receiving diaphragm 22 by the pressure of the fluid is pulled to the flow path 21 side by the pressure of the fluid, and the connecting part 23 pulls the connecting part 11, which is connected thereto by magnetic force, to the flow path 21 side. Accordingly, the pressure of the fluid is detected as a negative pressure by the diaphragm 12a. As described above, according to the pressure detection device 100 of the present embodiment, the pressure of a fluid can be accurately determined in both cases where the pressure of the fluid is a positive pressure and where the pressure of the fluid is a negative pressure while enhancing speed and safety of operation to change the fluid that is to be introduced to the flow path 21.

Further, according to the pressure detection device 100 of the present embodiment, the first joining face 23a1 of the joining member 23a joined to the pressure receiving diaphragm 22 and the pressure receiving diaphragm 22 are joined to each other by the adhesive agent AD in a predetermined range surrounding the center position P0. Since the joining member 23a and the pressure receiving diaphragm 22 are made of resin, respectively, the joining member 23a and the pressure receiving diaphragm 22 are joined to each other more firmly than in a case where the magnetic material 23b, which is a metal material, is joined to the pressure receiving diaphragm 22 by the adhesive agent. Thus, even when a change in the stress occurs in the joining portion between the connecting part 23 and the pressure receiving diaphragm 22, it is possible to maintain the state where the connecting part 23 is firmly joined to the pressure receiving diaphragm 22 and prevent the connecting part 23 joined to the pressure receiving diaphragm 22 of the flow path unit 20 from separating from the pressure receiving diaphragm 22.

Further, according to the pressure detection device 100 of the present embodiment, the second joining face 23a2 of the joining member 23a and the magnetic material 23b, which is a metal material, are joined to each other by the adhesive agent AD. Since the magnetic material 23b is made of metal while the joining member 23a is made of resin, the joining strength between the second joining face 23a2 and the magnetic material 23b is lower than in a case where resin members are joined to each other. In contrast, the metal material is not directly joined to the pressure receiving diaphragm 22, and a displacement of the second joining face 23a2 is small even when receiving a pressure of a fluid. It is thus possible to suitably maintain the state where the second joining face 23a2 and the magnetic material 23b, which is a metal material, are joined to each other by the adhesive agent AD.

According to the pressure detection device 100 of the present embodiment, an excessive portion of the adhesive agent AD applied to the first joining face 23a1 and the second joining face 23a2 can be accommodated in the communication through-hole 23a3, and this makes it possible to obtain a state where the first joining face 23a1 and the second joining face 23a2 are joined to each other by a suitable amount of the adhesive agent.

According to the pressure detection device 100 of the present embodiment, the first outer diameter D1 of the first joining face 23a1 is 1/3 times or greater of the second outer diameter D2 of the region of the pressure receiving diaphragm 22 with which a fluid comes into contact, and this can ensure a sufficient size of the first outer diameter D1 of the first joining face 23a1 joined to the pressure receiving diaphragm 22 and thereby ensure sufficient attractive force so that the connecting part 23 and the connecting part 11 are attracted to each other by magnetic force. Further, the first outer diameter D1 of the first joining face 23a1 is 1/2 times or less of the second outer diameter D2 of the region of the pressure receiving diaphragm 22 with which a fluid comes into contact, and this makes it possible to prevent the ability to follow a pressure change of the pressure receiving diaphragm 22 from compromising due to an excessively larger first outer diameter D1 of the first joining face 23a1.

According to the pressure detection device 100 of the present embodiment, the third outer diameter D3 of the connecting part 11 is larger than the first outer diameter D1 of the connecting part 23, and this makes it possible to ensure a sufficient third outer diameter D3 of the connecting part 11 and thereby ensure sufficient attractive force so that the connecting part 23 and the connecting part 11 are attracted to each other by magnetic force.

According to the pressure detection device 100 of the present embodiment, since the joining member 23a is joined to the magnetic material 23b with the protruding part 23b2 being inserted in the recess 23a2A, when joining the joining member 23a to the magnetic material 23b, it is possible to suitably prevent these members from being shifted in the radial direction RD orthogonal to the axis Y2 and enhance the joining strength of these members.

## Claims

1. A pressure detection device (100) comprising:
a pressure detection unit (10) configured to determine a pressure transferred to a pressure detection part;
a flow path unit (20) having a flow path formed in the flow path unit (20), a fluid being allowed to flow through the flow path; and
an attaching mechanism (30) configured to detachably attach the flow path unit (20) to the pressure detection unit (10),
wherein the pressure detection unit (10) includes
a pressure sensor (12) having the pressure detection part, and
a first connecting part (11) joined to the pressure detection part,
wherein the flow path unit includes
a resin pressure receiving part (22) configured to displace in response to receiving a pressure of a fluid flowing through the flow path, and
a second connecting part (23) joined to the pressure receiving part (22),
wherein either one of the first connecting part (11) and the second connecting part (23) includes a magnet, and the other of the first connecting part (11) and the second connecting part (23) includes a magnet or a magnetic material,
wherein in a state where the flow path unit (20) is attached to the pressure detection unit (10) by the attaching mechanism (30), the first connecting part (11) and the second connecting part (23) are arranged attracted to each other by magnetic force,
wherein the pressure receiving part (22) is formed circularly in planar view and formed in a plate shape from the center position to an end of the pressure receiving part (22),
wherein the second connecting part (23) includes a resin joining member (23a) formed cylindrically extending along the axis and having a first joining face and a second joining face formed at both ends in a direction along the axis, the first joining face being joined to the pressure receiving part (22), and the second joining face being joined to the magnet or the magnetic material, and
wherein the first joining face of the joining member (23a) and the pressure receiving part (22) are joined to each other by an adhesive agent in a predetermined range surrounding the center position, and the second joining face of the joining member (23a) and the magnet or the magnetic material are joined to each other by an adhesive agent.

2. The pressure detection device (100) according to claim 1, wherein the joining member (23a) includes a communication through-hole (23a3) arranged at the center position, the first joining face and the second joining face being in communication with each other through the communication through-hole (23a3).

3. The pressure detection device (100) according to claim 1, wherein a first outer diameter of the first joining face when viewed in planar view is set to be 1/3 times or greater and 1/2 times or less of a second outer diameter of a region of the pressure receiving part (22) when viewed in planar view, the fluid coming into contact with the region.

4. The pressure detection device (100) according to claim 1, wherein the outer diameter of the first connecting part (11) about the axis orthogonal to the pressure receiving part (22) is larger than the outer diameter of the second connecting part (23) about the axis.

5. The pressure detection device (100) according to any one of claims 1 to 4,
wherein the first joining face is a flat face arranged on a plane orthogonal to the axis,
wherein the second joining face is a stepwise face having a recess formed at the center position,
wherein the magnet or the magnetic material joined to the joining member (23a) includes a protruding part formed at the center position, and
wherein the joining member (23a) is joined to the magnet or the magnetic material with the protruding part being inserted in the recess.
